# EUROPEAN PATENT SPECIFICATION

(11) **EP 2 650 034 B1**
(45) Date of publication and mention of the grant of the patent: **15.05.2019**
(21) Application number: 13151190.9
(22) Date of filing: 14.01.2013
(51) Int. Cl.: A61M 16/12, B01F 3/02, B01F 5/04

(54) **Intake module**
Einlassmodul
Module d'admission

(30) Priority: 09.04.2012 TW 101112448
(43) Date of publication of application: 16.10.2013
(73) Proprietor: Galemed Corporation, I-Lan Hsien 26841 (TW)
(72) Inventor: Lee, Gary C. J., 26841 I-Lan Hsien (TW); Hsu, Ding-Yang, 11467 Taipei City (TW)
(74) Representative: Regimbeau

(56) References cited:
- EP-A1- 1 759 731
- WO-A2-02/074370
- US-A- 3 581 742
- US-A- 5 165 398
- US-A1- 2009 126 731

## Description

The invention relates to an intake module, more particularly to an intake module having an entrainment cover by which ambient air is entrained to increase the total flow rate of the intake module.

A current respiratory device typically includes a respiratory mask coupled to a compressed oxygen cylinder by an intake module for entraining ambient air into the compressed oxygen to dilute the oxygen concentration and increase the total flow rate of the gas flowing into the respiratory mask. However, the diversion mechanisms for entraining ambient air in current intake modules are unable to steadily entrain ambient air into the compressed oxygen stream, making the current mechanisms inefficient for increasing the total flow rate.

Document US 2009/126731 describes an entrainment cover adapted for use in an intake module. In that purpose, a patient interface assembly includes a housing that defines an inlet port and an outlet port. A jet pump receives pressurized gas flow from the inlet port and delivers the gas flow to the outlet port. A nebulizer is fluidly coupled to the outlet port and positioned to introduce medication into the gas flow and deliver medicated gas flow to a patient.

Document US 3 581 742 describes an apparatus for lung ventilation comprising a breathing manifold assembly, a venturi chamber body connected to said assembly having: an injector nozzle, a venturi throat, a venturi chamber behind said throat and around said nozzle, the position of said nozzle outlet relative to said throat to permit high initial flow rate, within the range of 25--90 liters/min. to said assembly, an intake check valve located in communication with said chamber to permit flow unidirectionally into said chamber, a variable size exhaust port to provide communication from the chamber to the exterior, means to supply fluid pressure to said nozzle inlet, and a flow control bypass valve connected to said inlet which, when closed, forces said fluid into said nozzle.

Document EP 1 759 731 describes a ventilatory support device with administration of gas having a predetermined content of oxygen in continuously positive pressure. The device comprises a pipe extending between an intake fitting and a delivery fitting of the gas, wherein an inner portion of the pipe diverges towards the delivery fitting. The device also comprises a substantially tubular injector supported in the pipe and orientated towards the delivery opening, and suitable for being connected to a pressurised gas source having a predetermined oxygen content.

Therefore, an object of the present invention is to provide an intake module for steadily and uniformly entraining ambient air into the compressed oxygen stream to increase the total flow rate of the gas flowing into the respiratory mask. The invention is defined by the independent claim 1.

According to the present invention, there is provided an intake module comprising an air delivery tube, an intake lid, an entrainment cover, and a needle tube.

The air delivery tube includes an outer tube body and an inner tube body disposed in the outer tube body. The outer tube body includes an intake end, an exhaust end opposite to the intake end, an intake chamber defined by the outer tube body, and an intake port that is formed between the intake end and the exhaust end and that exposes the intake chamber. The inner tube body extends from the exhaust end towards the intake end, and includes an exhaust hole disposed at an end thereof and adjoining the exhaust end, and an intake hole disposed at an opposite end thereof and in fluid communication with the intake chamber.

The intake lid covers the intake end, seals the intake chamber, and includes an inlet hole that extends through the intake lid and allows the intake chamber to be in fluid communication with the atmosphere. The entrainment cover is sleeved on the end of the inner tube body formed with the intake hole, and has a positioning hole formed therethrough and located between the intake hole and the inlet hole, and an entrainment port for establishing fluid communication between the intake chamber and the intake hole of the inner tube body. The needle tube establishes fluid communication between the inlet hole of the intake lid and the positioning hole of the entrainment cover, and defines a jet hole for spraying a compressed gas from the needle tube into the intake hole. The diameter of the jet hole is less than that of the intake hole of the inner tube body.

Preferably, the entrainment cover is a hollow and generally conical, and includes a securing ring portion that is sleeved fixedly on the inner tube body, a tapered portion that extends and tapers from the securing ring portion towards the intake end, and an end wall at a tip of the tapered portion. The entrainment port is formed in the tapered portion. The positioning hole is formed in the end wall. The entrainment cover defines a mixing space that is in fluid communication with the intake hole, the entrainment port, and the jet hole.

Additionally, the air delivery tube defines a central axis (L), about which the inner tube body and the jet hole are centered.

Preferably, the intake lid further includes a lid body sealably engaging the intake end of the outer tube body, and a rod extending from the lid body towards the inner tube body. The inlet hole extends through the rod and the lid body, is centered about the central axis (L), and has a first hole portion proximal to the lid body for is adapted to be in fluid communication with a compressed gas source, and a second hole portion distal from the lid body and having a diameter smaller than that of the first hole portion. The needle tube extends through the second hole portion.

According to an example, there is provided an entrainment cover by which ambient air can be steadily and uniformly entrained into the compressed gas stream to increase the total flow rate of the gas flowing into the respiratory mask.

The entrainment cover is a hollow and conical, is sleeved fixedly on the inner tube body, and has a positioning hole for establishing fluid communication between the intake hole and the inlet hole via the needle tube. The entrainment cover further has an entrainment port for establishing fluid communication between the intake chamber and the intake hole of the inner tube body, and defines a mixing space that is in fluid communication with the inlet hole, the entrainment port, and the jet hole.

Preferably, the entrainment cover further includes a securing ring portion sleeved fixedly on the inner tube body, a tapered portion extending and tapering from the securing ring portion towards the intake end, and an end wall at a tip of the tapered portion. The entrainment port is formed in the tapered portion. The positioning hole is formed in the end wall.

The efficacy of this invention resides in the needle tube and the entrainment cover. Specifically, the needle tube is fixed between the positioning hole of the entrainment cover and the inlet hole, so as to provide a steady stream of compressed gas into the intake module along the central axis. The entrainment cover is sleeved fixedly the inner tube body, and allows for steady and uniform entrainment of ambient air into the compressed gas stream provided by the needle tube to achieve an increased total flow rate of the gas flowing into the respiratory mask.

Other features and advantages of the present invention will become apparent in the following detailed description of a preferred embodiment with reference to the accompanying drawings, of which:
Figure 1 is an exploded perspective view of the preferred embodiment of an intake module of the present invention;
Figure 2 is an assembled perspective view of the preferred embodiment; and
Figure 3 is a sectional view of the preferred embodiment.

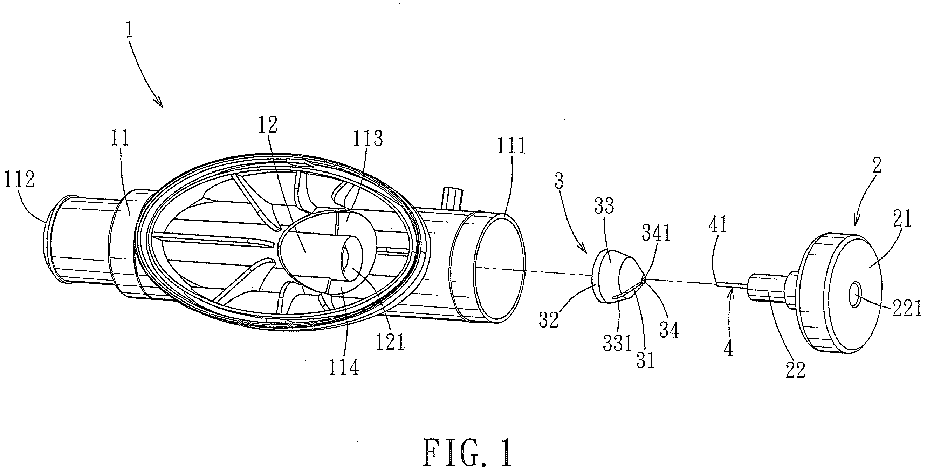

With reference to Figure 1, the preferred embodiment of an intake module of the present invention is coupled to a compressed oxygen cylinder (not shown) via a conduit tube (not shown), and is connected with a respiratory mask (not shown) via a flexible pipe (not shown). The intake module receives compressed oxygen from the compressed oxygen cylinder, and passes it through the flexible pipe and into the respiratory mask for inspiration by a user.

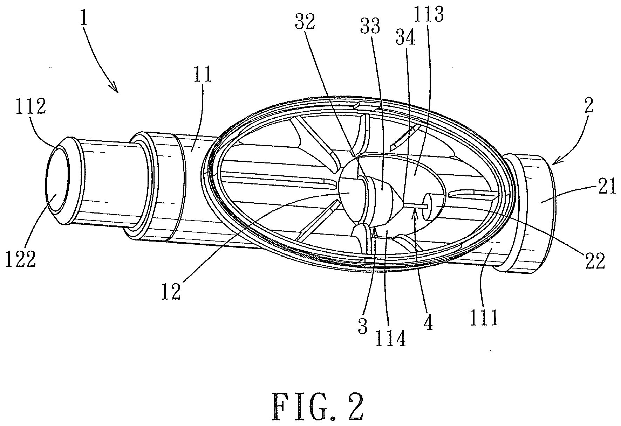

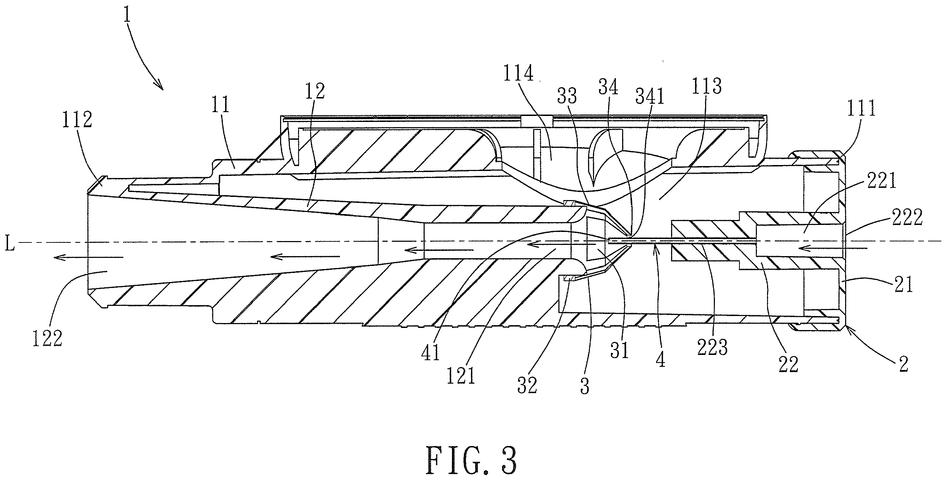

With additional reference to Figures 2 and 3, the intake module comprises an air delivery tube 1 having a central axis (L), an intake lid 2 sealably engaging an end of the air delivery tube 1, a hollow, generally conical entrainment cover 3 disposed within the air delivery tube 1, and a needle tube 4 for establishing fluid communication between the intake lid 2 and the air delivery tube 1.

The air delivery tube 1 includes an outer tube body 11 and an inner tube body 12 disposed in the outer tube body 11, both of which are centered about the central axis (L). The outer tube body 11 has an intake end 111 that is engageable with the intake lid 2, an exhaust end 112 opposite to the intake end 111, an intake chamber 113 defined by the outer tube body 11 and open at the intake end 111, and an intake port 114 that is formed in the outer tube body 11 between the intake end 111 and the exhaust end 112 and that exposes the intake chamber 113 to the atmosphere. The compressed oxygen flows into the air delivery tube 1 through the intake end 111 of the outer tube body 11. Ambient air flows into the intake chamber 113 through the intake port 114. The inner tube body 12 extends from the exhaust end 112 towards the intake end 111, and includes an exhaust hole 122 that is disposed at an end thereof and that is defined by the exhaust end 112, and an intake hole 121 that is disposed at an opposite end thereof and that is in fluid communication with the intake chamber 113.

The intake lid 2 includes a lid body 21 for sealably engaging the intake end 111 of the outer tube body 11, a rod 22 extending from the lid body 21 towards the inner tube body 12, and an inlet hole 221 extending through the rod 22 and the lid body 21 along the central axis (L). The inlet hole 221 has a first hole portion 222 proximate to the lid body 21, and a second hole portion 223 distal from the lid body 21. The diameter of the second hole portion 223 is less than that of the first hole portion 222.

The entrainment cover 3 includes a securing ring portion 32 securely sleeved on the end of the inner tube body 12 formed with the intake hole 121, a tapered portion 33 extending and tapering from the securing ring portion 32 towards the intake end 111, and an end wall 34 at a tip of the tapered portion 33. A positioning hole 341 is formed in the end wall 34 for receiving one end of the needle tube 4. An entrainment port 331 is formed in the tapered portion 33 and configured as a rectangular-cross-sectioned slot extending from the middle of the tapered portion 33 to the end wall 34 for establishing fluid communication between the intake chamber 113 and the intake hole 121 of the inner tube body 12. The size of the entrainment port 331 can be varied during manufacture of the entrainment cover 3 to adjust the amount of ambient air to entrain. In addition, the shape and number of the entrainment port 331 are not limited to those in the preferred embodiment and may be modified as necessary to achieve desired results.

Further, the entrainment cover 3 defines a mixing space 31 that is in fluid communication with a jet hole 41 of the needle 4, the entrainment port 331, and the intake hole 121 and that receives the compressed oxygen flowing from the jet hole 41 and ambient air flowing from the entrainment port 331 to decrease the concentration of oxygen, so as to direct the mixture of the compressed oxygen with ambient air into the intake hole 121.

The jet hole 41 has a diameter less than those of the conduit tube and the intake hole 121 of the inner tube body 12 for spraying the compressed oxygen from the needle tube 4 into the mixing space 31. In this example, one end of the needle tube 4 is inserted through the second hole portion 223 of the inlet hole 221, while the other end is inserted through the positioning hole 341 and into the fixedly extends along the central axis (L).

Operation of the intake module of the present invention requires the conduit tube to be disposed between and in fluid communication with the compressed oxygen cylinder and the first hole portion 222 of the inlet hole 221 so that the compressed oxygen flows through the inlet hole 221 out of the jet hole 41 of the needle tube 4. Because the diameter of the conduit tube is greater than that of the jet hole 41, the velocity of the compressed oxygen increases and the pressure of the compressed oxygen decreases as it flows through the jet hole 41. The jet hole 41 directs the flow of compressed oxygen into the mixing space 31 of the entrainment cover 3 towards the intake hole 121 of the inner tube body 12. Specifically, when the mixing space 31 receives the stream of compressed oxygen from the jet hole 41, the compressed oxygen stream flows at high velocity and low pressure compared to the ambient air within the mixing space 31. Via the Venturi effect, the pressure difference causes the stream of compressed oxygen to entrain ambient air in the mixing space 31 as it flows into the intake hole 121.

The entrainment of ambient air into the compressed oxygen results in a decrease in the oxygen concentration of the gas flowing through the mixing space 31, and an increase in the gas within the stream. This mixture flows through the inner tube body 12 and out of the intake module from the exhaust hole 122 into the flexible pipe.

Importantly, the entrainment cover 3 firmly secures the inner tube body 12 and the needle tube 4 along the central axis (L) for optimally introducing ambient air into the intake hole 121 of the inner tube body 12 via the Venturi effect. The compressed oxygen stream steadily and uniformly entrains ambient air, incorporating it therein while flowing to the intake hole 121, thus increasing the total flow rate of the gas flowing from the intake module into the respiratory mask.

Further, due to the Venturi effect, when ambient air from the mixing space 31 is entrained by the compressed oxygen into the intake hole 121, the air pressure within the mixing space 31 decreases. The resulting pressure difference causes the air outside of the outer tube body 11 to enter the intake chamber 113 through the intake port 114. This ambient air flows from the intake port 114 into the mixing space 31 through the entrainment port 331 to replenish the mixing space 31 with air.

To sum up, the fixed position of the needle tube 4 between the inlet hole 221 of the intake lid 2 and the positioning hole 341 of the entrainment cover 3 along the central axis (L) allows the compressed oxygen to flow into the mixing space 31 and, via the Venturi effect, steadily and uniformly entrain ambient air as it flows through the mixing space 31. The addition of ambient air into the compressed oxygen stream increases the total flow rate and thus fulfills the purpose of this invention.

## Claims

1. An intake module comprising:
an air delivery tube (1) including an outer tube body (11) and an inner tube body (12) disposed in said outer tube body (11), said outer tube body (11) having an intake end (111), an exhaust end (112) opposite to said intake end (111), an intake chamber (113) that is defined by said outer tube body (11) and that is open at said intake end (111), and an intake port (114) that is formed between said intake end (111) and said exhaust end (112) and that exposes said intake chamber (113), said inner tube body (12) extending from said exhaust end (112) towards said intake end (111) and having an exhaust hole (122) disposed at an end thereof and adjoining said exhaust end (112), and an intake hole (121) disposed at an opposite end thereof and in fluid communication with said intake chamber (113); and
an intake lid (2) covering said intake end (111) and sealing said intake chamber (113), said intake lid (2) having an inlet hole (221) adapted for allowing said intake chamber (113) to be in fluid communication with the outside therethrough so that a compressed gas is fed into said inlet hole (221);
**characterized by**
an entrainment cover (3) sleeved on the end of said inner tube body (12) formed with said intake hole (121) and having a positioning hole (341) formed therethrough and disposed between said inlet hole (221) and said intake hole (121), and an entrainment port (331) disposed between and in fluid communication with said intake chamber (113) and said intake hole (121); and
a needle tube (4) extending into said inlet hole (221) and said positioning hole (341) and defining a jet hole (41) adapted for spraying the compressed gas from said needle tube (4) into said intake hole (121) in said inner tube body (12) of said air delivery tube (1), said jet hole (41) having a diameter less than that of said intake hole (121) of said inner tube body (12);
wherein, when the compressed gas is delivered from said intake lid (2) into said inner tube body (12) of said air delivery tube (1), air is drawn into said intake hole (121) to mix with the compressed gas according to Venturi effect.

2. The intake module in claim 1, **characterized in that** said entrainment cover (3) is hollow and generally conical, and defines a mixing space (31) in fluid communication with said intake hole (121), said entrainment port (331), and said jet hole (41).

3. The intake module in claim 2, **characterized in that** said entrainment cover (3) has a securing ring portion (32) sleeved fixedly on said inner tube body (12), a tapered portion (33) extending and tapering from said securing ring portion (32) toward said intake end (111), and an end wall (34) disposed at a tip of said tapered portion (33), said entrainment port (331) being formed in said tapered portion (33), said positioning hole (341) being formed of said end wall (34).

4. The intake module in any one of claims 1 to 3, **characterized in that** said air delivery tube (1) defines a central axis, said inner tube body (12) and said jet hole (41) being centered about said central axis.

5. The intake module in claim 4, **characterized in that** said intake lid (2) includes a lid body (21) engaging said outer tube body (11) and a rod (22) extending from said lid body (21) toward said intake hole (121) of said inner tube body (12), said inlet hole (221) extending along said central axis through said rod (22) and said lid body (21), said inlet hole (221) having a first hole portion (222) proximal to said lid body (21) along said central axis and a second hole portion (223) distal from said lid body (21) along said central axis, said second hole portion (223) having a diameter smaller than that of said first hole portion (222), said needle tube (4) extending through said second hole portion (223).

## Patentansprüche

1. Einlassmodul, aufweisend:
ein Luftzufuhrrohr (1), aufweisend einen äußeren Rohrkörper (11) und einen inneren Rohrkörper (12), der im äußeren Rohrkörper (11) angeordnet ist, wobei der äußere Rohrkörper (11) ein Einlassende (111), ein dem Einlassende (111) gegenüber liegendes Auslassende (112), eine durch den äußeren Rohrkörper (11) definierte und am Einlassende (111) offene Einlasskammer (113), und eine zwischen dem Einlassende (111) und dem Auslassende (112) ausgebildete Einlassöffnung (114), die die Einlasskammer (113) freilegt, wobei sich der innere Rohrkörper (12) vom Auslassende (112) in Richtung des Einlassendes (111) erstreckt und ein an einem Ende davon angeordnetes und an das Auslassende (112) angrenzendes Auslassloch (122) aufweist, und ein Einlassloch (121), das an einem gegenüberliegenden Ende davon angeordnet ist und in Fluidverbindung mit der Einlasskammer (113) steht; und
einen Einlassdeckel (2), der das Einlassende (111) bedeckt und die Einlasskammer (113) abdichtet, wobei der Einlassdeckel (2) ein Einlassloch (221) aufweist, das so angepasst ist, dass es der Einlasskammer (113) eine Fluidverbindung hindurch mit der Außenseite gestattet, so dass ein komprimiertes Gas in das Einlassloch (221) eingeleitet wird;
**gekennzeichnet durch**
eine Mitnahmeabdeckung (3), die, auf das Ende des inneren Rohrkörpers (12) geschoben, mit dem Einlassloch (121) ausgebildet ist und ein durchgehendes Positionierungsloch (341) aufweist, und das zwischen dem Einlassloch (221) und dem Einlassloch (121) angeordnet ist, und eine Mitnahmeöffnung (331), die zwischen der Einlasskammer (113) und dem Einlassloch (121) angeordnet ist und in Fluidverbindung mit dieser steht; und
ein Nadelrohr (4), das sich in das Einlassloch (221) und das Positionierungsloch (341) erstreckt und ein zum Sprühen des komprimierten Gases aus dem Nadelrohr (4) in das im inneren Rohrkörper (12) des Luftzufuhrrohrs (1) angeordneten Einlasslochs (121) ausgebildetes Düsenloch (41) definiert, wobei das Düsenloch (41) einen Durchmesser aufweist, der kleiner ist als der des Einlasslochs (121) des inneren Rohrkörpers (12);
wobei, wenn das komprimierte Gas vom Einlassdeckel (2) in den inneren Rohrkörper (12) des Luftzufuhrrohrs (1) geliefert wird, Luft in das Einlassloch (121) gesaugt wird, um sich gemäß dem Venturi-Effekt mit dem komprimierten Gas zu vermischen.

2. Einlassmodul nach Anspruch 1, **dadurch gekennzeichnet, dass** die Mitnahmeabdeckung (3) hohl und allgemein konisch ist und einen Mischraum (31) definiert, der in Fluidverbindung mit dem Einlassloch (121), der Mitnahmeöffnung (331) und dem Düsenloch (41) steht.

3. Einlassmodul nach Anspruch 2, **dadurch gekennzeichnet, dass** die Mitnahmeabdeckung (3) einen Sicherungsringabschnitt (32) aufweist, der fest auf den inneren Rohrkörper (12) geschoben ist, einen sich verjüngenden Abschnitt (33), der sich verjüngend vom Sicherungsringabschnitt (32) in Richtung zum Einlassende (111) erstreckt und eine Endwand (34), die an einer Spitze des sich verjüngenden Abschnitts (33) angeordnet ist, wobei die Mitnahmeöffnung (331) in dem sich verjüngenden Abschnitt (33) ausgebildet ist, wobei das Positionierungsloch (341) von der Endwand (34) gebildet wird.

4. Einlassmodul nach einem der Ansprüche 1 bis 3, **dadurch gekennzeichnet, dass** das Luftzufuhrrohr (1) eine zentrale Achse definiert, wobei der innere Rohrkörper (12) und das Düsenloch (41) um die zentrale Achse herum zentriert sind.

5. Einlassmodul nach Anspruch 4, **dadurch gekennzeichnet, dass** der Einlassdeckel (2) einen Deckelkörper (21) umfasst, der mit dem äußeren Rohrkörper (11) in Eingriff steht, und eine Stange (22), die sich vom Deckelkörper (21) zum Einlassloch (121) des inneren Rohrkörpers (12) erstreckt, wobei sich das Einlassloch (221) entlang der zentralen Achse durch die Stange (22) und den Deckelkörper (21) erstreckt, wobei das Einlassloch (221) einen ersten Lochabschnitt (222) proximal zum Deckelkörper (21) entlang der zentralen Achse und einem zweiten Lochabschnitt (223) distal vom Deckelkörper (21) entlang der zentralen Achse aufweist, wobei der zweite Lochabschnitt (223) einen Durchmesser aufweist, der kleiner ist als der des ersten Lochabschnitts (222), wobei sich das Nadelrohr (4) durch den zweiten Lochabschnitt (223) erstreckt.

## Revendications

1. Module d'admission comprenant :
un tube de distribution d'air (1) comprenant un corps de tube externe (11) et un corps de tube interne (12) disposé dans ledit corps de tube externe (11), ledit corps de tube externe (11) ayant une extrémité d'admission (111), une extrémité d'échappement (112) opposée à ladite extrémité d'admission (111), une chambre d'admission (113) qui est définie par ledit corps de tube externe (11) et qui est ouverte au niveau de ladite extrémité d'admission (111), et un orifice d'admission (114) qui est formé entre ladite extrémité d'admission (111) et ladite extrémité d'échappement (112) et qui expose ladite chambre d'admission (113), ledit corps de tube interne (12) s'étendant à partir de ladite extrémité d'échappement (112) vers ladite extrémité d'admission (111) et ayant un trou d'échappement (122) disposé au niveau de son extrémité et attenant à ladite extrémité d'échappement (112) et un trou d'admission (121) disposé au niveau de son extrémité opposée et en communication de fluide avec ladite chambre d'admission (113) ; et
un couvercle d'admission (2) recouvrant ladite extrémité d'admission (111) et scellant ladite chambre d'admission (113), ledit couvercle d'admission (2) ayant un trou d'entrée (221) adapté pour permettre à ladite chambre d'admission (113) d'être en communication de fluide avec l'extérieur à travers ce dernier de sorte qu'un gaz comprimé est amené dans ledit trou d'entrée (221) ;
**caractérisé par** :
un couvercle d'entraînement (3) emmanché sur l'extrémité dudit corps de tube interne (12) formé avec ledit trou d'admission (121) et ayant un trou de positionnement (341) formé à travers ce dernier et disposé entre ledit trou d'entrée (221) et ledit trou d'admission (121), et un orifice d'entraînement (331) disposé entre et en communication de fluide avec ladite chambre d'admission (113) et ledit trou d'admission (121) ; et
un tube d'aiguille (4) s' étendant dans ledit trou d'entrée (221) et ledit trou de positionnement (341) et définissant un trou de jet (41) adapté pour pulvériser le gaz comprimé à partir dudit tube d'aiguille (4) dans ledit trou d'admission (121) dans ledit corps de tube interne (12) dudit tube de distribution d'air (1), ledit trou de jet (41) ayant un diamètre inférieur à celui dudit trou d'admission (121) dudit corps de tube interne (12) ;
dans lequel, lorsque le gaz comprimé est distribué dudit couvercle d'admission (2) dans ledit corps de tube interne (12) dudit tube de distribution d'air (1), l'air est aspiré dans ledit trou d'admission (121) pour se mélanger avec le gaz comprimé selon l'effet Venturi.

2. Module d'admission selon la revendication 1, **caractérisé en ce que** ledit couvercle d'entraînement (3) est creux et généralement conique, et définit un espace de mélange (31) en communication de fluide avec ledit trou d'admission (121), ledit orifice d'entraînement (331) et ledit trou de jet (41).

3. Module d'admission selon la revendication 2, **caractérisé en ce que** ledit couvercle d'entraînement (3) a une partie de bague de fixation (32) emmanchée de manière fixe sur ledit corps de tube interne (12), une partie progressivement rétrécie (33) s'étendant et se rétrécissant progressivement de ladite partie de bague de fixation (32) vers ladite extrémité d'admission (111), et une paroi d'extrémité (34) disposée au niveau d'une pointe de ladite partie progressivement rétrécie (33), ledit orifice d'entraînement (331) étant formé dans ladite partie progressivement rétrécie (33), ledit trou de positionnement (341) étant formé avec ladite paroi d'extrémité (34).

4. Module d'admission selon l'une quelconque des revendications 1 à 3, **caractérisé en ce que** ledit tube de distribution d'air (1) définit un axe central, ledit corps de tube interne (12) et ledit trou de jet (41) étant centrés autour dudit axe central.

5. Module d'admission selon la revendication 4, **caractérisé en ce que** ledit couvercle d'admission (2) comprend un corps de couvercle (21) mettant en prise ledit corps de tube externe (11) et une tige (22) s'étendant à partir dudit corps de couvercle (21) vers ledit trou d'admission (121) dudit corps de tube interne (12), ledit trou d'entrée (221) s'étendant le long dudit axe central à travers ladite tige (22) et ledit corps de couvercle (21), ledit trou d'entrée (221) ayant une première partie de trou (222) à proximité dudit corps de couvercle (21) le long dudit axe central et une seconde partie de trou (223) à distance dudit corps de couvercle (21) le long dudit axe central, ladite seconde partie de trou (223) ayant un diamètre inférieur à celui de ladite première partie de trou (222), ledit tube d'aiguille (4) s'étendant à travers ladite seconde partie de trou (223).
